# EUROPEAN PATENT APPLICATION

(11) **EP 1 536 364 A2**
(43) Date of publication of application: **01.06.2005**
(21) Application number: 04257108.3
(22) Date of filing: 17.11.2004
(51) Int. Cl.: G06F 19/00

(54) **Bio-information presenting device and bio-information presenting method**

(30) Priority: 27.11.2003 JP 2003398154
(71) Applicant: Sony Corporation, Tokyo 141-0001 (JP)
(72) Inventor: Miyajima, Yasushi, c/o Sony corporation, Tokyo (JP); Sako, Yoichiro, c/o Sony corporation, Tokyo (JP); Terauchi, Toshiro, c/o Sony corporation, Tokyo (JP); Inoue, Makoto, c/o Sony corporation, Tokyo (JP); Asukai, Masamichi, c/o Sony corporation, Tokyo (JP); Shirai, Katsuya, c/o Sony corporation, Tokyo (JP); Takai, Motoyuki, c/o Sony corporation, Tokyo (JP); Makino, Kenichi, c/o Sony corporation, Tokyo (JP)
(74) Representative: Smith, Samuel Leonard

(57) **Abstract**

A bio-information presenting method and a bio-information presenting device, for presenting the bio-information, indicating the movement of a physical organ of a user, for the user, are disclosed. The movement of a bodily organ of the user, controlled voluntarily or involuntarily, is fed back to the user. A background image is stored in a storage unit for image data for composition 5. An image processing unit 6 compounds an image of the user, captured by a camera 8, with a background image. In compounding the images, the image of the user is subjected to fade-in and fade-out in association with an output of a breathing sensor 7. The user is apprized of the inhalation period, exhalation period and the depth of breathing, based on an image demonstrated on a display unit 10. The change in the display image surface produces certain changes in the user's breathing. The display unit 10 of the breathing visualizing device 1 is changed responsive to the breathing of the user. A feedback loop is formed in this manner between the breathing visualizing device 1 and the respiratory organ of the user.

## Description

This invention provides a bio-information presenting method and a bio-information presenting device for presenting the bio-information, indicating the movement of a physical organ of a user, for the user.

This application claims priority of Japanese Patent Application No. 2003-398154, filed on November 27, 2003, the entirety of which is incorporated by reference herein.

The respiratory organ is controlled by the respiratory center in a brain stem of the brain and is in operation without dependency on the user's intention. The respiratory center checks whether or not oxygen producing the energy for activating the cells of the human body is sufficient, and whether or not carbon dioxide generated is being discharged correctly, and issues a command for strengthening or weakening the respiratory muscles.

On the other hand, the muscle used for respiration is the voluntary muscle that may be moved by the user's will, and hence the human being is able to control the respiration based on the own will. Conscious breathing is said to be correlated with the control of the soul and the body. It is said that, in dancing, as an example, dancer's movements are smoothed by making a swinging movement during the exhalation period, and may be stopped in an unforced fashion by halting the breathing. In the martial arts, a larger power than that usually produced may be produced during the exhalation period. Moreover, the art of breathing is needed for performance of instruments, training for swimming and elocution, or for training of yoga or kiko healing.

Since the movements of the respiratory organ can hardly be checked from outside, it is necessary for the human being to sensually recognize movements of the own abdominal part or chest part, during training in breathing, in order to verify in person, based on the recognized results, whether the correct breathing method is being observed. However, judgment based on sensual recognition lacks in objectiveness.

There has so far been known a reciprocal feedback system in which the bio-information is fed back as sound. However, this system consists in two persons exchanging their own bio-information in real-time in order to promote understanding of each other. With this system, the user is unable to recognize the movement of his or her own respiratory organ.

An example of a known system is disclosed in Japanese Patent Application Laid-Open No. 2001-129093

It is an object of the present invention to provide a bio-information presenting device and a bio-information presenting method whereby the movements of a user's physical organ, controlled voluntarily or non-voluntarily, may be fed back to the user.

In one aspect, the present invention provides a bio-information presenting device comprising detection means for detecting a movement of physical organs, controlled involuntarily or voluntarily, as the bio-information, presenting means for presenting the information, and presenting contents controlling means for controlling the contents presented by the presenting means, based on the bio-information detected by the detection means.

In another aspect, the present invention provides a method for presenting the bio-information comprising a detection step of detecting a movement of a physical organ, controlled involuntarily or voluntarily, as the bio-information, a presentation contents generating step of generating presented contents, indicating the movement of the physical organ, based on the bio-information, and a presenting step of presenting the presented contents to a user.

According to the present invention, in which the movements of the physical organ, controlled voluntarily or involuntarily, are visualized, the evaluation of the training in a movement is no longer based on sensual judgment but is based on objective judgment. Moreover, a trainee may be conscious of involuntary control by the autonomic nerve, so that the present method and device may be used for entertainment purposes by enabling the control of the unconscious movement of the human body.

Embodiments of the invention will now be described, by way of example only, with reference to the accompanying drawings in which:
Fig.1 is a schematic view showing a feedback loop formed between a user and a breathing visualizing device.
Fig.2 is a block diagram showing the structure of the breathing visualizing device.
Fig.3 is a graph showing an output level of a breathing sensor.
Fig.4 is flowchart showing the sequence of the image composition processing.
Fig.5 schematically shows α-blending processing.
Fig.6 shows composite images referenced to output levels of the breathing sensor.
Fig.7 is a flowchart for illustrating the operation of the breathing visualizing device.
Fig.8 is a flowchart showing the processing sequence in a first picture outputting method.
Fig.9 shows an example of an output image in the first picture outputting method.
Fig.10 is a flowchart showing the processing sequence in a second picture outputting method.
Fig.11 shows an example of an output image in the second picture outputting method.
Fig.12 shows an example of an output image in a third picture outputting method.
Fig.13 shows an example of an output image on which is superimposed the information pertinent to the breathing.
Fig. 14 is a block diagram showing the structure of a personal computer in a third embodiment.

Referring now to the drawings, a breathing visualizing device 1 of the present invention is explained in detail. The breathing visualizing device generates and demonstrates an image indicating movements of the respiratory organs of a user. The user is able to recognize movements of their own respiratory organs from demonstrated contents of the breathing visualizing device.

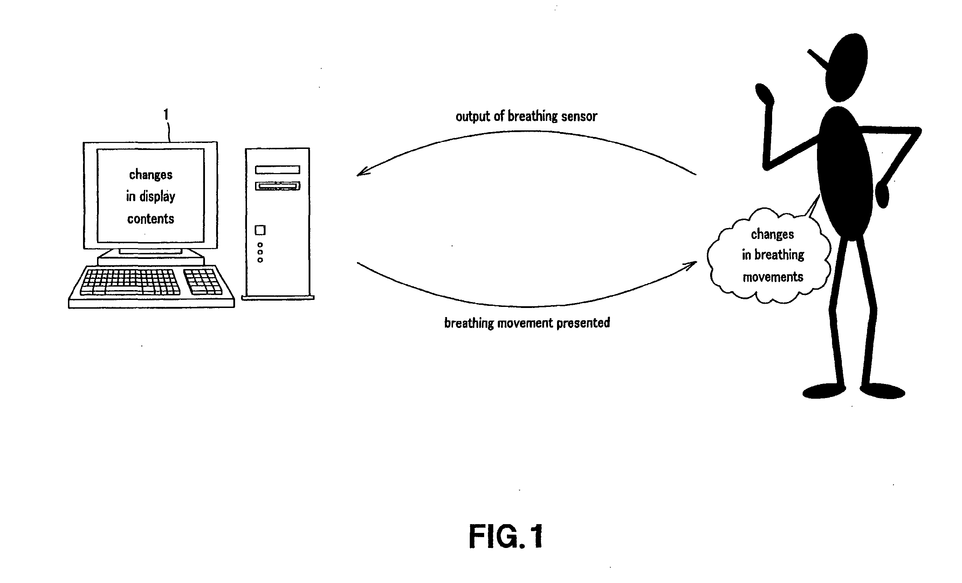

The breathing visualizing device 1 and the user's respiratory organs form a feedback system shown in Fig.1. That is, when the user breathes, the display image of the breathing visualizing device 1 is changed. The changes in the demonstrated image on the breathing visualizing device 1 produce some changes in the user's breathing. The demonstrated image on the breathing visualizing device 1 is changed responsive to changes in the user's breathing. The user becomes aware of the movements of his/her respiratory organs, of which the user is usually unconscious, and thus consciously controls the movements of the respiratory organ.

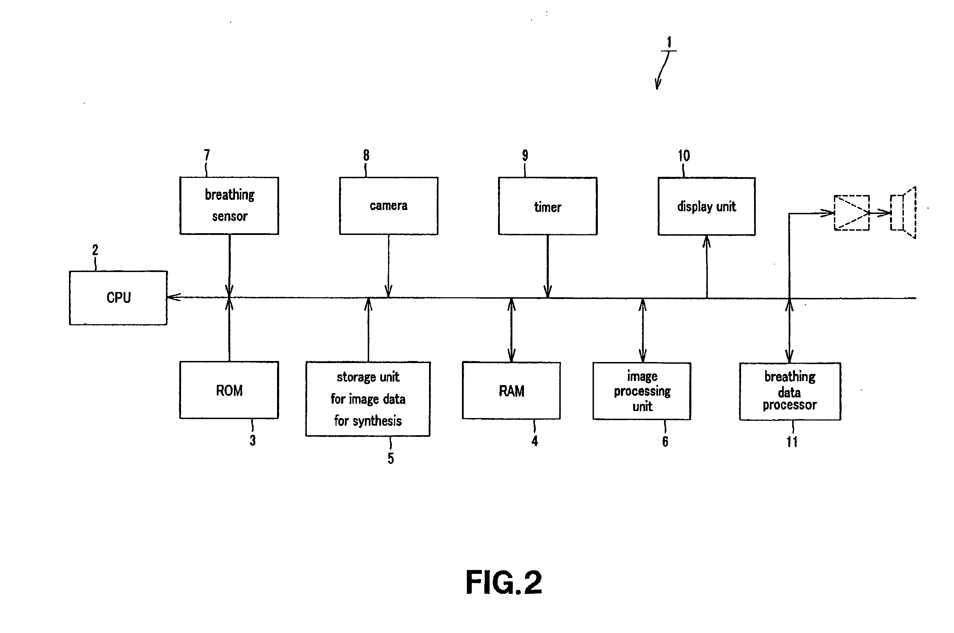

Fig.2 is a block diagram showing the structure of the breathing visualizing device 1. A storage unit for image data for composition 5 has stored therein an image of the user, captured by a camera 8, and a background image, in an image processing unit 6. In compounding the images, the user's image is subjected to fade-in or fade-out in association with an output of a breathing sensor 7. The so generated image is output to a display unit 10. A CPU (central processing unit) 2 causes the programs or the setting information, stored in a ROM (read-only memory) 3, to be extended in a RAM (random access memory) 4, to control the breathing visualizing device 1.

The breathing sensor 7 is usually an extensible belt wound about a breast or abdominal part. The breathing sensor 7, wound about the breast part, detects the costal breathing, while the breathing sensor 7, wound about the abdominal part, detects the abdominal breathing. The breathing sensor 7 is expanded and contracted in association with the user's breathing. The breathing sensor 7 is formed of a material. the electrical resistance of which changed in proportion to the depth of breathing. The higher an output level of the breathing sensor 7, the more is the extension of the material of the breathing sensor 7, that is, the more is the amount of air inhaled in breathing. In the present description, the amount of air inhaled into the user's body is indicated as the depth of breathing. The depth of breathing is correlated with the output level of the breathing sensor 7.

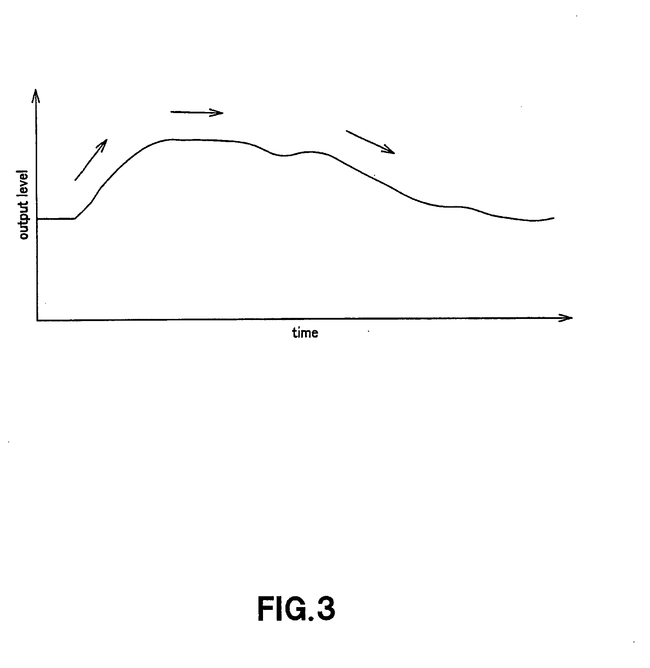

Fig.3 is a graph showing an output level of the breathing sensor 7. In Fig.3, the ordinate and the abscissa denote the output level of the breathing sensor 7 and time, respectively. When the user is inhaling and exhaling air, the output of the breathing sensor 7 is increased and decreased gradually, respectively. When the user is not inhaling air nor exhaling air, the output level is constant. In the present description, the domain with a positive gradient is termed an inhalation period, the domain with a negative gradient is termed an exhalation period, and the domain with a constant output is termed a non-breathing period.

A breathing data processor 11 increments the number of the exhalation periods (number of times of exhalation) if, in the exhalation period, the output level becomes minimum. The breathing data processor 11 increments the number of the inhalation periods (number of times of inhalation) if, in the inhalation period, the output level becomes minimum. Moreover, the breathing data processor 11 causes a storage part to store the time point of incrementing the number of times of exhalation and the time point of incrementing the number of times of inhalation.

The time as from the time point of incrementing the number of times of exhalation until the time point of incrementing the number of times of inhalation next time corresponds to the user's breathing period. The number of times of exhalation or that of inhalation per unit time represents the number of times of breathing cycles per unit time. The breathing data processor 11 counts the number of times of exhalation or that of inhalation up to a time point one minute before the current time.

The image processing unit 6 superimposes a background image and a user's image, captured by the camera 8, one on the other. An image generated by the image processing unit 6 is changed in association with the user's breathing. In this case, the user's image undergoes fade-in or face-out in association with the user's breathing.

The image processing unit 6 processes the background image and the user's image with α-blending. This α-blending is the image processing of summing a semi-transparent image to a background image. An α-value indicates the degree of transparency of an image and, the higher the α-value, the higher becomes the transparency of the image. The α-value assumes the value of 0 to 1. With the α-value of 0, the user's image is clear. The user's image becomes progressively transparent, with increase in the α-value, and vanishes with the α-value equal to unity. With α-blending, fade-in and fade-out of the user's image may be achieved by converting the α-value.

The image processing unit 6 calculates the α-value, while compounding the images. In calculating the α-value, an output level of the breathing sensor 7 when the α-value is 1 or 0 is set. In setting the output level, calibration may be carried out at the time of loading the breathing sensor 7, or the user's breathing quantity may be checked by the image processing unit 6 by learning. The image processing unit 6 calculated the α-value in meeting with the output level of the breathing sensor 7, so that the α-value will be equal to 0 to 1.

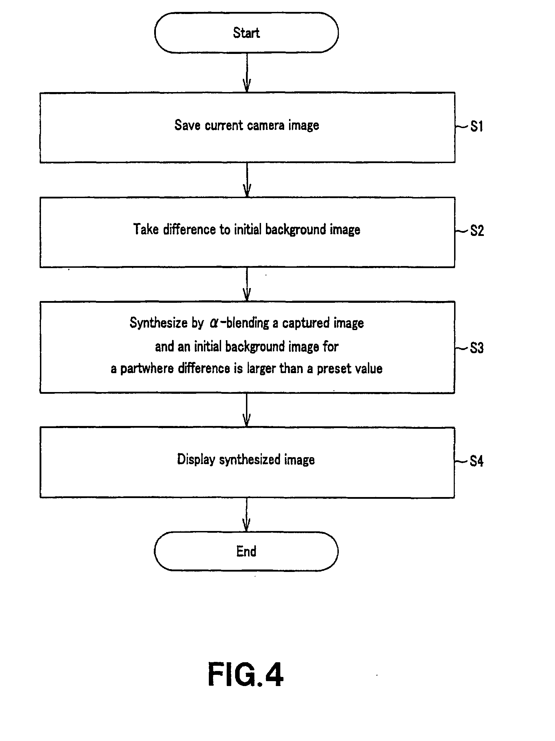

The image processing unit 6 compounds an image using the calculated α-value. Fig.4 depicts a flowchart showing the sequence of operations for image composition processing by the image processing unit 6. First, the user's image, captured by the camera 8, is saved in the storage unit for image data for composition 5 (step S1) The image processing unit 6 takes a difference between the image captured by the camera 8 and the background image to extract an image only of the user. The background image is stored in advance in the storage unit for data for composition. This image has been captured by the camera when the user is not there (step S2).

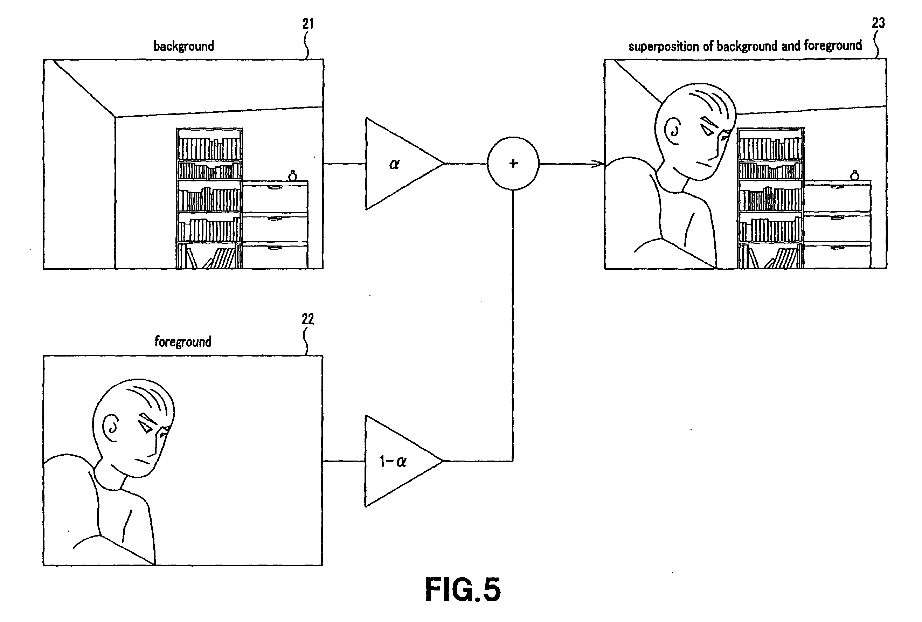

The image processing unit 6 compounds the background image and the image only of the user. Fig.5 schematically shows the processing of the α-blending processing in the image processing unit 6. In Fig.5, an upper left image is a background image 21, and a lower left image is an image 22 corresponding to the captured image less the background. The image processing unit 6 sums the background image 21, stored in the storage unit for image data for composition, and multiplied with the degree of transparency α, to a user's image 22, captured by the camera 8, and multiplied with (1-α), to generate a composite image 23 (step S3). The image processing unit 6 causes the composite image 23 to be displayed on the display unit 10 (step S4).

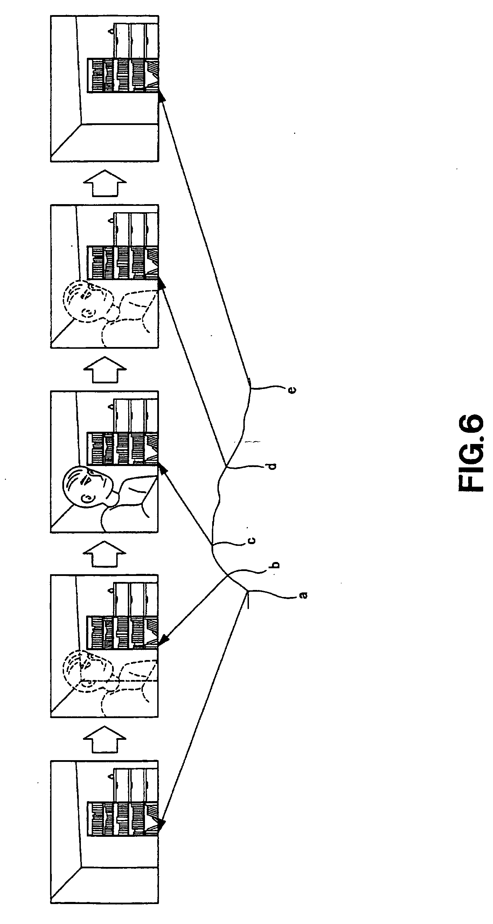

Fig.6 shows an example of an image generated by the image processing unit 6. In a lower part of Fig.6, there is shown a graph indicating an output level of the breathing sensor 7 since the user begins to inhale until he/she has finished inhalation. In an upper part of Fig.6, there are shown images generated by the image processing unit 6. A point a denotes an output level before the user begins to inhale. At this time, there is no air inhaled in the user's lung. The α-value is unity (1), there being no user's image displayed.

In a point b, the user inhales a minor amount of air, with the output level of the breathing sensor 7 becoming higher. The α-value becomes smaller in inverse proportion to the output level of the breathing sensor 7. If the α-value becomes smaller, the user's image becomes clear. In a point c, the user inhales a sufficient amount of air, with the output level of the breathing sensor 7 becoming locally maximum. At this time, the α-value is zero, and the user's image is displayed clearly.

When the user begins to exhale air, the output level of the breathing sensor 7 becomes smaller. The α-value is progressively increased in inverse proportion thereto and, at a point d, the user's image is displayed semi-transparently. At a point e where the output level of the breathing sensor 7 is minimum, the user's image ceases to be displayed.

It is also possible with the image processing unit 6 to generate an image changed with a delay with respect to an output of the breathing sensor 7. For generating a delayed image, an output of the breathing sensor 7 is transiently stored in a RAM. After lapse of the delay time, the output of the breathing sensor 7 is read out and the α-value corresponding to this output is calculated and demonstrated on the display surface.

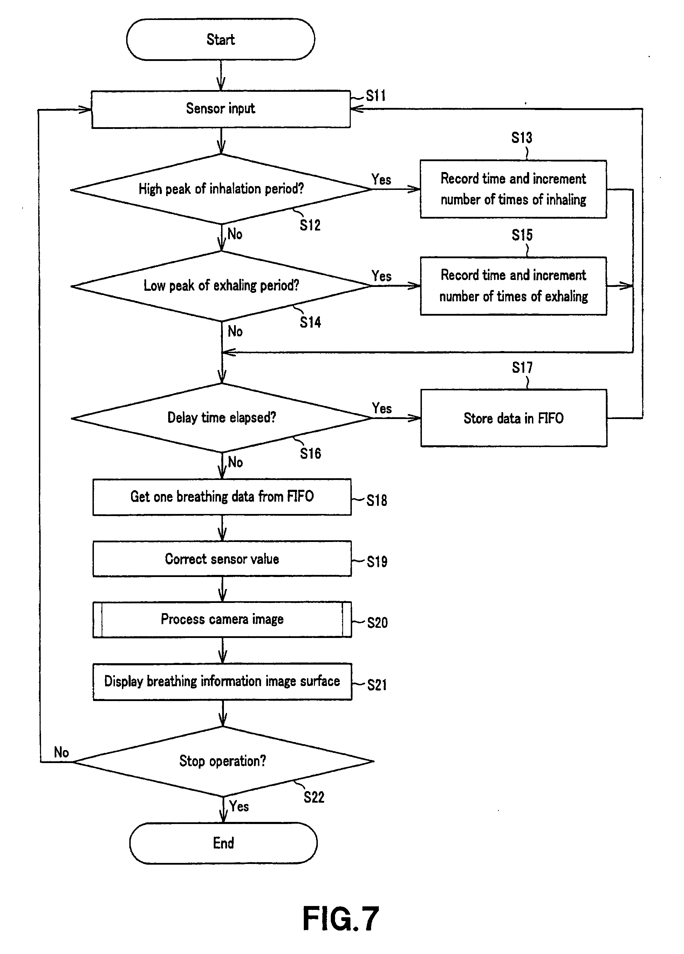

Referring to Fig.7, the operation of the breathing visualizing device 1, having the above-described structure, is explained. In this processing, an image delayed from the breathing movement is generated.

First, the breathing sensor 7 detects the bio-information, exhibiting the breathing movement, and outputs the detected results to the main body unit of the breathing visualizing device 1 (step S11). When the output level of the breathing visualizing device 1 is increasing or decreasing, the breathing data processor 11 deems that the user is inhaling or exhaling, respectively. When the user is inhaling, the breathing data processor 11 awaits until the output level of the breathing sensor 7 is maximum (step S12; NO). When the output level of the breathing sensor 7 is maximum (step S12; YES), the breathing data processor 11 increments the number of times of inhalation and causes the time point of incrementing to be stored in a RAM (step S13).

If, on the other hand, the output level of the breathing sensor 7 is decreasing, the breathing data processor 11 deems that the user is exhaling. When the user is exhaling, the breathing data processor 11 awaits until the output level of the breathing sensor 7 is minimum (step S14; NO). When the output level of the breathing sensor 7 is minimum (step S14; YES), the breathing data processor 1lincrements the number of times of inhalation and causes the time point of incrementing to be stored in the RAM (step S15).

The image processing unit 6 verifies whether or not the delay time has elapsed and, if the delay time has not elapsed (step S16; NO), the image processing unit 6 causes the output of the breathing sensor 7 to be stored in the RAM. In this case, the image processing unit 6 causes an output of the breathing sensor 7 to be stored in accordance with a FIFO (first-in first-out) system (step S17). After storage of the output of the breathing sensor 7, the image processing unit 6 shits the processing to a step S11.

If, on the other hand, the delay time has elapsed (step S16; YES), the image processing unit 6 takes out one data from the FIFO (step S18) and corrects the output level of the breathing sensor 7 to a value suited to the user's bodily constitution (step S19).

The image processing unit 6 processes the background image, stored in the storage unit for image data for composition, and the user's image, captured by the camera 8, with α-blending. Since the value of this α-blending is changed in inverse proportion to the depth of breathing of the user, the user's image becomes thicker or thinner in keeping with the depth of breathing (step S20).

The image processing unit 6 outputs the composite image to the display unit 10 (step S21). The breathing visualizing device 1 then detects whether or not a stop command for image display has been entered from the user. In case the stop command has been entered (step S22; YES), image display comes to a close. If conversely the stop command has not been entered (step S22; NO), the image processing unit shifts the processing to the step S11.

In this manner, the breathing visualizing device 1 generates an image indicating the breathing movements of the user, such as the depth of breathing or the breathing period, and demonstrates the so generated image on the display unit 10. By checking this image, the user is able to recognize his/her breathing.

Moreover, with the present breathing visualizing device 1, in which the real-time image of the user, captured by the camera 8, undergoes fade-in or fade-out, the process of the user's image appearing and then disappearing is repeated in keeping with the user's breathing, and hence the impact given by the image is strong, so that the user is obliged to be more conscious of his/her own breathing.

### Example 1

The breathing visualizing device 1 may be applied to a device for entertainment whereby the user may objectively check breathing movements occurring in his/her own body. Or, the breathing visualizing device may be used as a training device for improving the breathing habit. In case the breathing visualizing device 1 is used for training, it may be used in combination with a bio-information sensor, different from the breathing sensor 7, for more efficient training.

For example, in breathing arts, practiced from old, such as in yoga or in martial arts, abdominal breathing, by up-and-down movement of the diaphragm, is recommended. Air may be inhaled with the nose and exhaled with vibrations of the vocal tract. One may put most of efforts into one's lower abdominal part to one's waist part in order to stabilize one's waist part.

An utmost concentration power is needed if one is to be conscious of plural body sites, such as diaphragm, vocal tract or muscles of the lower abdominal part. With the breathing visualizing device 1 for training, plural sensors may be mounted to sites of which one has to be conscious to permit the user to check whether or not the breathing is being made in a correct fashion.

The breathing visualizing device 1 is provided, as bio-information sensors, with breathing sensors 7, mounted to the abdominal part and the chest part, an EMG (Electro-Myography) sensor, mounted to the abdominal part, and a vibration sensor, mounted to a throat. The breathing sensors 7 measure the abdominal breathing and costal breathing simultaneously to detect whether the user is breathing by the abdominal breathing. The EMG sensor, mounted to the abdominal part, detects whether or not the user is putting utmost effort to the muscle of the abdominal part, while the vibration sensor, mounted to the vocal tract, detects whether or not the vocal tract is in a vibrating state. Based on the results detected by these bio-information sensors, the breathing visualizing device 1 is able to verify which part is to be trained. The breathing visualizing device 1 may present the sites in need of training to the user to guide the user to learn the correct breathing habit.

In many cases, the training in breathing is carried out as the trainee is in meditation. On the other hand, if the breathing training practice is taken into dance or sports, a trainee cannot view an image display surface. Hence, the breathing visualizing device 1, designed for training, may output the breathing movements by voice or vibrations, so that a user who has closed his/her eye or who is moving his/her body may be apprised of his/her breathing movements. When the breathing movement is output as voice, the beep sound may be produced in timed relation to inhalation or exhalation, the sound interval or volume of the speech signals may be varied, or the site in need of training may be indicated by speech, such as "put efforts into lower abdominal part". If the speech output is made in a location where there are plural users, it is preferred to use different speech signals from user to user to provide an accurate feedback system. In case of outputting with vibrations, these vibrations are output in timed relation to the inhalation or exhalation periods.

It is noted that the mounting positions of the bio-information sensors or the output of the breathing visualizing device 1 may be changed depending on the training methods for the breathing practice. The breathing method may be classed into a method of exhaling air from one's nose and a method of exhaling air from one's mouth. With these methods, the output of the breathing visualizing device 1 is reversed, even though the sorts or the mounting positions of the bio-information sensors remain the same.

### Example 2

In the Example 2, three different image outputting methods are explained. In a first image outputting method, the display image surface in its entirety is faded-in or faded-out in association with output levels of the breathing sensor 7.

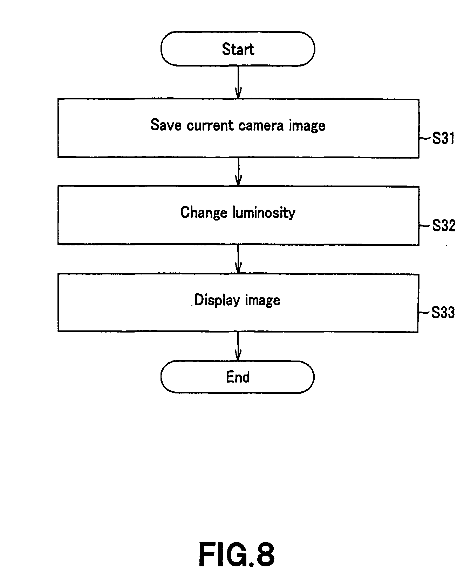

Fig.8 depicts a flowchart showing a sequence of processing operations of the image processing unit 6. First, a user's image, captured by the camera 8, is saved in the storage unit for image data for composition 5 (step S31). The image processing unit 6 is supplied with an output of the breathing sensor 7, as an input, and calculates a value multiplied with luminosity of image data, based on an output of the breathing sensor 7. The calculating method is now explained. The image processing unit 6 first empirically learns the maximum value of the output level, and calculates the proportion of the current output level for the maximum value of the output level equal to unity (1). This proportion value is the value multiplied by luminosity (step S32).

This value is proportional to the quantity of air inhaled by a user. When a user has inhaled or inhaled a sufficient quantity of air, this value is 1 or 0, respectively. The image demonstrated on the display unit 10 undergoes fade-out and fade-in as this value is decreased and increased, respectively (step S33).

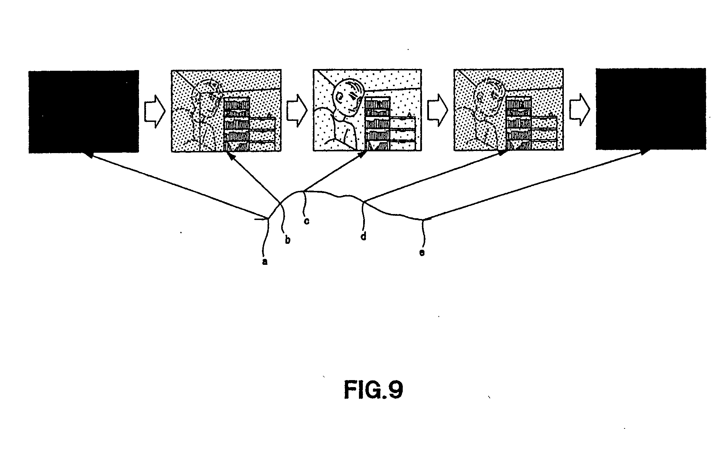

Fig.9 shows exemplary outputs in the first image outputting method, and specifically shows image changes since the time the user begins inhalation until he/she has finished exhalation. Before the user begins inhalation, the output level of the breathing sensor 7 is low, so that the value multiplied by luminosity is 0. Hence, an image with the luminosity equal to 0, that is, a black image, is displayed. During the time the user is inhaling, the value multiplied by the luminosity assumes a value between 0 and 1, and hence an image lower in luminosity than the original image is displayed. When the user has inhaled a sufficient quantity of air, the value multiplied by the luminosity is 1, and hence an image of the same luminosity as the original image is displayed. When the user exhales, the luminosity of the image displayed is gradually lowered. When the user has finished exhalation, a black image is displayed.

The second image outputting method is now explained. This second image outputting method consists in switching between two images. It is assumed that one of the two images is an image captured by the camera 8 and the other is an entirely black image. The two images may be different from these images, that is, may be different from the image captured by the camera and the black image.

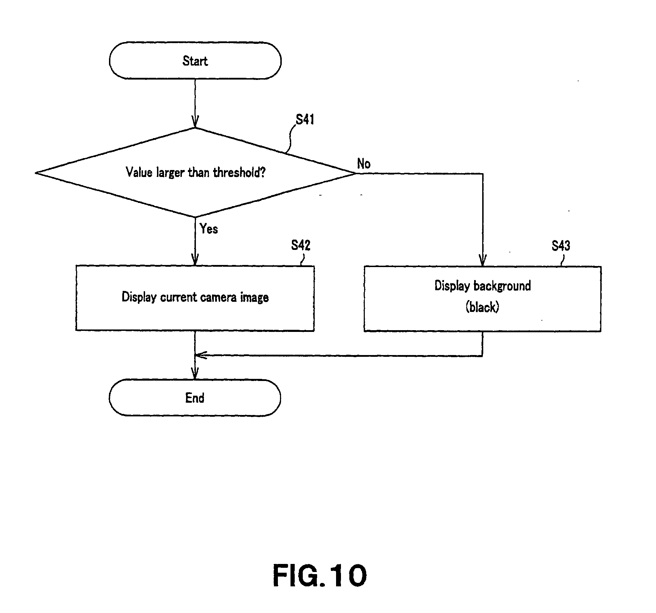

With the second image outputting method, a certain threshold value is set in advance of image outputting. The threshold value may be entered manually, or determined by learning by the image processing unit 6. Fig.10 shows the sequence of operations of the image processing unit 6 in the second image outputting method. The image processing unit 6 compares the output level of the breathing sensor 7 to the threshold value. When the output level of the breathing sensor 7 is larger than the threshold value (step S41; YES), the image captured by the camera 8 is output to the display unit 10 (step S42). If, on the other hand, the output level of the breathing sensor 7 is smaller than the threshold value (step S41; NO), a black image is demonstrated on the display unit 10 (step S43).

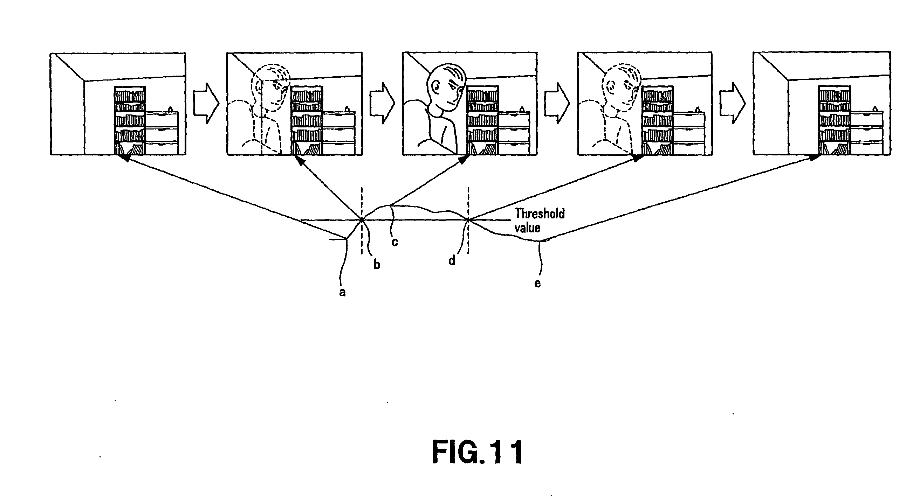

Fig.11 shows exemplary outputs in the second image outputting method. In a lower part of Fig.11, there is shown a graph indicating an output level of the breathing sensor 7. A horizontal reference line, entered in the graph, denotes the height of the threshold value. A point of intersection of the output level of the breathing sensor 7 and the threshold value is the image switching timing. When the output level of the breathing sensor 7 is higher than the threshold value, the image from the camera 8 is output to the display unit 10 and, when the output level of the breathing sensor 7 is lower than the threshold value, the black image is demonstrated on the display unit 10.

The third image outputting method varies the background of an output image, as introduced in a beginning part of the explanation of the preferred embodiments. In the above-described image outputting method, an image devoid of a user is captured by the camera 8 and a difference between an image being captured by the camera 8 and the image devoid of the user is taken to generate an image only of the user. The image only of the user and the image devoid of the user are subjected to α-blending to generate an image in which only the user undergoes fade-in and fade-out.

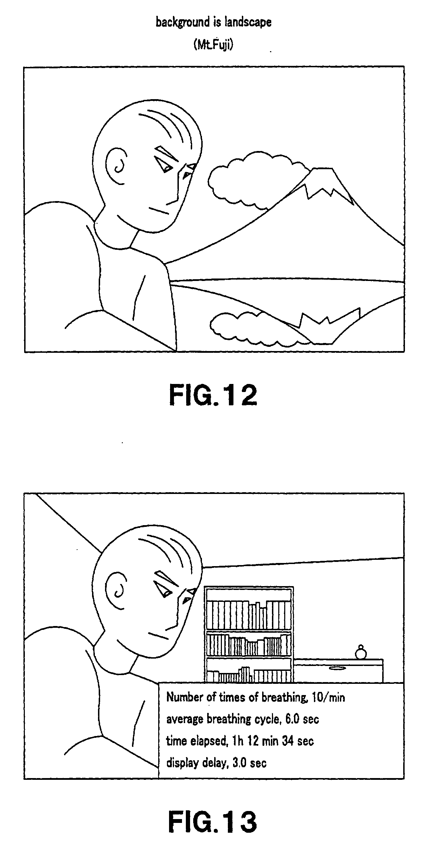

With the third image outputting method, an image different from the image is used as a background image at the time of the α-blending. Fig.12 shows an output obtained on α-blending an image of Mt.Fuji and the user's image. Not only the background but also a forefront image may be changed. For example, an animation may be compounded in place of the user's image.

The image composition unit is also able to compound the information pertinent to the breathing, as calculated by the breathing data processor 11. Fig. 13 shows an exemplary image in which the information pertinent to breathing is displayed in superposition. The image composition unit demonstrates e.g. the breathing per unit time, as calculated by the breathing data processor 11, breathing period, time elapsed as from the time an image has commenced to be displayed, and delay time, in superposition on the display unit 10. In this manner, the breathing visualizing device 1 is able to provide the user with the accurate and detailed breathing information.

### Example 3

A device for visualizing movements of an organ other than the respiratory organs is now explained. The present invention envisages to visualize the movements of the physical organs subjected to involuntary control by the autonomic nerve and to voluntary control by the user's will. The movements of bodily organs, subjected to voluntary control and to involuntary control, may be enumerated by eye blinking, eye-ball movements and EMG.

The eye blinking forms a tear film on the eyeball surface or protects the eyeballs from impacts. The eye blinking, for protecting the eyeballs from impacts, is the reflective movement and instantaneous. On the other hand, the eye blinking for forming a tear film on the eyeball surface is a continuous movement and, if the eye blinking is not carried out periodically, the eye tends to be dried.

Of these days, a term 'dry eye' has come to be used. As this term implies, the number of times of eye blinking of a user of a personal computer or a game player is decreased, such that the eye of the user of the personal computer or the game player tends to be dried. The Example 3 introduces a display image surface controlling method which will induce eye blinking of the user or player. The display image surface controlling method is applicable not only to a personal computer but also to an electronic apparatus having an image display surface, such as a game machine or a television receiver.

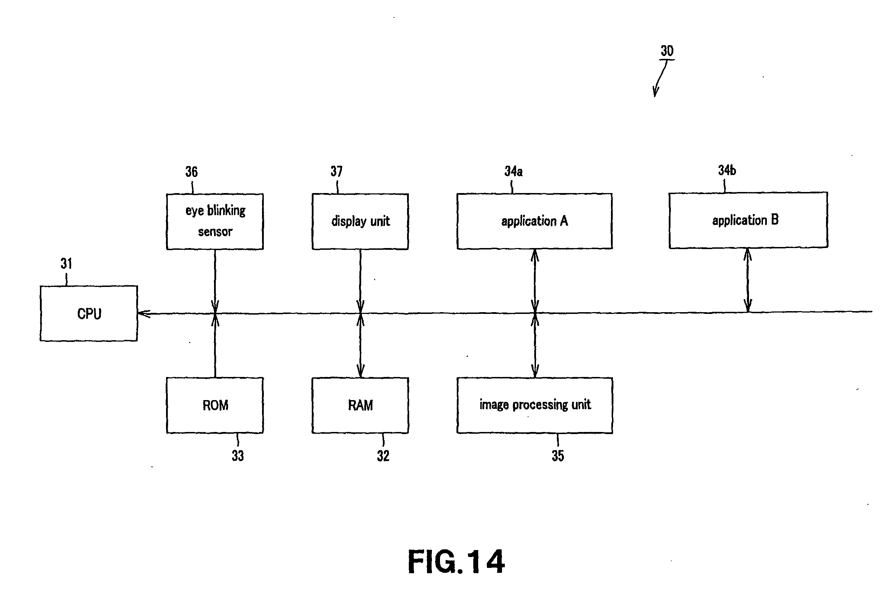

Fig.14 depicts a block diagram showing an inner structure of a personal computer 30. The personal computer 30 includes a CPU 31, as a calculation controlling device, a RAM 32, as a work area of the CPU 31, a ROM 33 for storage of a non-rewritable program or the setting information, applications 34a, 34b for executing various functions of the computer 30, an image processing device 35 for varying the display of the application image display surface responsive to the interval of eye blinking of the user, and an eye blinking sensor 36 for detecting the eye blinking of the user.

The image processing device 35 measures the time duration as from the time of a user's eye blinking until the user's next eye blinking. If eye blinking does not occur for longer than a predetermined time, the image processing device 35applies preset image processing on an image output to a display unit 37 in order to induce the user's eye blinking. This image processing includes blurring or darkening the displayed image, whereby the user becomes conscious of the shortage in the number of times of eye blinking to make conscious effort to make eye blinking.

## Claims

1. A bio-information presenting device comprising:
detection means for detecting a movement of a physical organ, controlled involuntarily or voluntarily, as the bio-information;
presenting means for presenting information; and
presenting contents controlling means for controlling contents of the information presented by said presenting means, based on the bio-information detected by said detection means.

2. The bio-information presenting device according to claim 1, wherein said bio-information is at least one of breathing movements, eyeball movements, eye blinking and electro-myography.

3. The bio-information presenting device according to claim 1 or 2, wherein said presenting means presents information pertinent to the bio-information to a user by using at least one of image, speech, light and vibrations.

4. The bio-information presenting device according to claim 1, 2 or 3, wherein said movement is a breathing movement and wherein said presenting contents controlling means causes said presenting means to present whether a user's respiratory organ is inhaling air or exhaling air.

5. The bio-information presenting device according to claim 4, wherein said presenting means is image display means and wherein said presenting contents control means causes said presenting means to demonstrate an image changed in association with air inhalation and air exhalation by the respiratory organ.

6. The bio-information presenting device according to claim 5, wherein a change in said image is delayed with respect to air inhalation and air exhalation by the respiratory organ.

7. The bio-information presenting device according to claim 5 or 6, wherein said image is an image of at least a part of a body of the user.

8. The bio-information presenting device according to claim 5, 6 or 7, wherein said image is the face of the user.

9. The bio-information presenting device according to any one of claims 5 to 8, wherein said presenting contents controlling means causes fade-in and fade-out of said image in association with air inhalation and air exhalation by said respiratory organ.

10. The bio-information presenting device according to any one of claims 5 to 9, wherein said presented image is made up by a background image and an image of the user; and wherein
said presenting contents controlling means varies the composition ratio of an image of the user in association with air inhalation and exhalation by said respiratory organ.

11. A method for presenting bio-information comprising:
a detection step of detecting a movement of a physical organ, controlled involuntarily or voluntarily, as the bio-information;
a presentation contents generating step of generating presented contents, indicating the movement of the physical organ, based on said bio-information; and
a presenting step of presenting said presented contents to a user.

12. The method for presenting bio-information according to claim 11, wherein the movement of a respiratory organ, detected by said detection step, is presented to a user.

13. The method for presenting bio-information according to claim 11 or 12, wherein said presented contents indicate whether the respiratory organ of a user is inhaling air or exhaling air.

14. The method for presenting bio-information according to claim 13, wherein said presentation contents generating step generates an image changed in association with air inhalation and exhalation by said respiratory organ.

15. The method for presenting bio-information according to claim 14, wherein a change in said image is delayed with respect to air inhalation and air exhalation by the respiratory organ.

16. The method for presenting bio-information according to claim 15, further comprising
a delay time setting step of having the user set the delay time.

17. The method for presenting bio-information according to claim 14, 15 or 16, wherein said image is an image of at least a part of a body of the user.

18. The method for presenting bio-information according to any one of claims 14 to 17, wherein said image is the face of the user.

19. The method for presenting bio-information according to any one of claims 14 to 18, wherein said presenting contents controlling step causes fade-in and fade-out of said image in association with air inhalation and air exhalation by said respiratory organ.

20. The method for presenting bio-information according to any one of claims 14 to 19, wherein said presented image is made up by a background image and an image of the user; and wherein
said presenting contents controlling step varies the composition ratio of an image of the user in association with air inhalation and exhalation by said respiratory organ.

21. The method for presenting bio-information according to claim 11, wherein said bio-information is at least one of eyeball movements, eye blinking and electro-myography.

22. The method for presenting bio-information according to claim 12, 13 or 21, wherein said presenting step presents the information pertinent to the bio-information to the user using at least one of image, speech, light and vibrations.
